# EUROPÄISCHE PATENTSCHRIFT

(11) **EP 1 056 343 B1**
(45) Veröffentlichungstag und Bekanntmachung des Hinweises auf die Patenterteilung: **13.10.2004**
(21) Anmeldenummer: 99906223.5
(22) Anmeldetag: 10.02.1999
(51) Int. Cl.: A01N 51/00, A01N 61/00

(54) **DERMAL APPLIZIERBARE WASSERHALTIGE FORMULIERUNGEN VON PARASITIZIDEN**
AQUEOUS FORMULATIONS OF PARASITICIDES FOR SKIN APPLICATION
FORMULATIONS AQUEUSES A APPLICATION CUTANEE DE PARASITICIDES

(30) Priorität: 23.02.1998 DE 19807633
(43) Veröffentlichungstag der Anmeldung: 06.12.2000
(73) Patentinhaber: Bayer HealthCare AG, 51368 Leverkusen (DE)
(72) Erfinder: SIRINYAN, Kirkor, D-51467 Bergisch Gladbach (DE); DORN, Hubert, D-42115 Wuppertal (DE); HEUKAMP, Ulrich, D-51515 Kürten (DE)
(86) Internationale Anmeldenummer: PCT/EP1999/000875
(87) Internationale Veröffentlichungsnummer: WO 1999/041986

(56) Entgegenhaltungen:
- DE-A- 4 443 888
- DE-A- 19 540 948
- DE-A- 19 543 477
- DE-A- 19 613 334

## Beschreibung

Die vorliegende Erfindung betrifft wasserhaltige Formulierungen zur dermalen Bekämpfung von parasitierenden Insekten an Tieren mittels Agonisten oder Antagonisten der nicotinergen Acetylcholinrezeptoren von Insekten.

Agonisten oder Antagonisten der nicotinergen Acetylcholinrezeptoren von Insekten sind bekannt. Zu ihnen gehören die Nicotinyl-Insektizide und ganz besonders die Chlornicotinyl-Insektizide. Ihre Anwendung gegen Flöhe ist bekannt z.B. aus WO 93/24002 und EP-A 682 869.

DE-A-44 43 888 offenbart Formulierungen für Agonisten oder Antagonisten der nicotinergen Acetylcholinrezeptoren von Insekten, die jedoch kein Wasser enthalten.

Es w urden nun neue wasserhaltige Formulierungen zur dermalen Anwendung von Agonisten oder Antagonisten der nicotinergen Acetylcholinrezeptoren von Insekten gefunden, die sich besonders zur dermalen Bekämpfung parasitierender Insekten wie Flöhe, Läuse oder Fliegen an Tieren eignen und sich durch ihre hervorragende Lagerstabilität bei tiefen Temperaturen (bis zu -30°C) auszeichnen.

Agonisten oder Antagonisten der nicotinergen Acetylcholinrezeptoren von Insekten sind bekannt z.B. aus Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389; Deutsche Offenlegungsschriften Nr. 3 639 877, 3 712 307; Japanische Offenlegungsschriften Nr. 03 220 176, 02 207 083, 63 307 857, 63 287 764, 03 246 283, 04 9371, 03 279 359, 03 255 072; US-Patentschriften Nr. 5 034 524, 4 948 798, 4 918 086, 5039686, 5 034 404; PCT-Anmeldungen Nr. WO 91/17 659, 91/4965; Französische Anmeldung Nr. 2 611 114; Brasilianische Anmeldung Nr. 88 03 621.

Die Erfindung betrifft wasserhaltige Formulierungen zur dermalen Bekämpfung von parasitierenden Insekten an Tieren der folgenden Zusammensetzung:
a) Imidacloprid in einer Konzentration von 1 bis 20 Gew.-% bezogen auf das Gesamtgewicht der Formulierung;
b) Wasser in einer Konzentration von 2,5 bis 15 Gew.-%, bezogen auf das Gesamtgewicht der Formulierung;
c) Lösungsmittel aus der Gruppe Alkohole oder gegebenenfalls substituierten Pyrrolidone in einer Konzentration von mindestens 20 Gew.-% bezogen auf das Gesamtgewicht der Formulierung;
d) Lösungsmittel aus der Gruppe cyclischer Carbonate oder Lactone in einer Konzentration von 5 bis 50 Gew.-% bezogen auf das Gesamtgewicht der Formulierung;
e) gegebenenfalls weitere Hilfsmittel aus der Gruppe Verdickungsmittel, Spreitmittel, Farbstoffe, Antioxidantien, Treibmittel, Konservierungsstoffe, Haftmittel, Emulgatoren;
f) gegebenenfalls weitere Wirkstoffe,
wobei die Summe von Wirkstoffen, Lösungsmitteln und Hilfsstoffen 100 Gew.-% beträgt.

Imidacloprid:

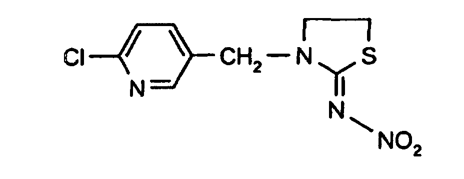

Die erfindungsgemäßen Formulierungen enthalten den Wirkstoff in Konzentrationen von 0,1 bis 20 Gew.-%, bevorzugt von 1 bis 12,5 Gew.-%.

Im allgemeinen hat es sich als vorteilhaft erwiesen, Mengen von etwa 0,5 bis etwa 50 mg, bevorzugt 1 bis 20 mg, Wirkstoff je Körpergewicht pro Tag zur Erzielung wirksamer Ergebnisse zu verabreichen.

Die Formulierung enthält 2,5 bis 15 Gew.-% Wasser, bevorzugt sind 4 bis 8 Gew.-%, besonders bevorzugt sind ca. 5 Gew.-% Wasser. Durch Zusatz des Wassers wird überraschenderweise eine beträchtliche Verbesserung der Kältestabilität der Formulierung gegen Ausfällen des Wirkstoffs bei niedrigen Temperaturen erzielt.

Als Lösungsmittel kommen in Frage:

Alkohole wie Benzylalkohol oder Tetrahydrofurfurylalkohol oder gegebenenfalls substituierte Pyrrolidone wie Pyrrolidon-2, 1-(C₂₋₂₀-Alkyl)-pyrrolidon-2, insbesondere 1-Ethylpyrrolidon, 1-Octylpyrrolidon, 1-Dodecylpyrrolidon, 1-Isopropylpyrrolidon, 1-(s.- oder t.- oder n-Butyl)-pyrrolidon, 1-Hexylpyrrolidon, 1-(C₂₋₁₀-Alkenyl)-pyrrolidon-2 wie 1-Vinylpyrrolidon-2, 1-(C₃₋₈-Cycloalkyl)-pyrrolidon-2 wie 1 -Cyclohexylpyrrolidon, 1-(C₁₋₆,-Hydroxyalkyl)-pyrrolidon-2, 1-(C₁₋₆-Alkoxy-C₁₋₆-alkyl)-pyrrolidon-2 wie 1-(2-Hydroxyethyl)-pyrrolidon, 1-(3-Hydroxypropyl)-pyrrolidon, 1-(2-Methoxyethyl)-pyrrolidon, 1-(3-Methoxypropyl)-pyrrolidon, ferner 1-Benzylpyrrolidon. Besonders genannt sei Benzylalkohol. Diese Lösungsmittel werden in Mischung mit weiteren Lösungsmitteln (Colösungsmitteln) eingesetzt.

Sie liegen vor in einer Konzentration von mindestens 40 Gew -%. bevorzugt 40 bis 85 Gew.-%, besonders bevorzugt 50 bis 80 Gew.-%.

Als Colösungsmittel kommen in Frage cyclische Carbonate oder Lactone. Als solche seien genannt: Ethylencarbonat, Propylencarbonat, γ-Butyrolacton.

Sie liegen vor in einer Konzentration von 5,0 bis zu 50 Gew.-%, bevorzugt von 7,5 bis 50 Gew -%. besonders bevorzugt von 10 bis 50 Gew -%.

Die Summe von Wirkstoffen, Losungsmitteln und Hilfsstoffen muß 100 Gew -% betragen.

Als weitere Hilfsmittel kommen in Frage: Konservierungsmittel wie Benzylalkohol (nicht erforderlich falls bereits als Lösungsmittel vorhanden), p-Hydroxybenzoesäureester, n-Butanol.

Verdickungsmittel wie: Anorganische Verdickungsmittel wie Bentonite, kolloidale Kieselsäure, Aluminiummonostearat, organische Verdickungsmittel wie Cellulosederivate, Polyvinylalkohole, Polyvinylpyrrolidone und deren Copolymere, Acrylate und Methacrylate.

Als Farbstoffe seien genannt alle zur Anwendung am Tier zugelassenen Farbstoffe, die gelöst oder suspendiert sein können.

Nilfsstoffe sind auch spreitende Öle wie Adipinsäure-di-2-ethylhexylester, Isopropylmyristat, Dipropylenglykolpelargonat, cyclische und acyclische Silikonöle wie Dimetikone und ferner deren Co- und Terpolymerisate mit Ethylenoxid, Propylenoxid und Formalin, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol und Vitamin E.

Ihre Menge kann im Bereich 0,01 bis 5,0 % (bezogen auf die gesamte Formuliermasse) breit variiert werden, wobei die Mengen zwischen 0,05 bis 3,0 zu bevorzugen sind. Die besonders bevorzugten Mengen liegen im Bereich 0,075 bis 2,5 %. Bevorzugte Antioxidantien sind Buthylhydroxytoluol, Tocophenol und Vitamin E.

Lichtschutzmittel sind z.B. Stoffe aus der Klasse der Benzophenone oder Novantisolsaure.

Haftmittel sind z. B Cellulosederivate, Starkederivate, Polyacrylate, naturliche Polymere wie Algmate, Gelatine.

Hilfsstoffe sind auch Emulgatoren wie nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphor-säureester-monoethanolaminsalz;
kationaktive Tenside wie Cetyltrimethylammoniumchlorid.

Weitere Hilfsstoffe sind Mittel mit denen die erfindungsgemäßen Formulierungen auf die Haut gesprüht oder gespritzt werden können. Dabei handelt es sich um die für Spraydosen benötigten üblichen Treibgase wie Propan. Butan, Dimethylether, CO₂ oder halogenierte Niedrigalkane, bzw. deren Mischungen untereinander.

Die erfindungsgemäßen Formulierungen eignen sich bei günstiger Warmblütoxizität zur Bekämpfung von parasitierenden Insekten, die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Zu den Schädlingen gehören:

Aus der Ordnung der Anoplura z.B. Haematopinus spp., Linognathus spp., Solenopotes spp., Pediculus spp., Pthims spp.;
aus der Ordnung der Mallophaga z.B. Trimenopon spp.. Menopon spp . Eomenacanthus spp. Menacanthus spp., Trichodectes spp., Felicola spp . Damalinea spp., Bovicola spp;
aus der Ordnung der Diptera z.B Chrysops spp., Tabanus spp., Musca spp . Hydrotaea spp., Muscina spp., Haematobosca spp., Haematobia spp., Stomoxys spp. Fannia spp., Glossina spp., Lucilia spp., Calliphora spp., Auchmeromyia spp., Cordylobia spp., Cochliomyia spp., Chrysomyia spp., Sarcophaga spp.,Wohlfartia spp., Gasterophilus spp., Oesteromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp..

Besonders hervorgehoben sei die Wirkung gegen Siphonaptera, insbesondere gegen Flöhe.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner. Gänse. Puten, Enten.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

In den erfindungsgemäßen Formulierungen können auch weitere Wirkstoffe enthalten sein. Zu den weiteren Wirkstoffen gehoren Insektizide wie phosphorhaltige Verbindungen, d.h. Phosphor- oder Phosphonsäureester, natürliche oder synthetische Pyrethroide, Carbamate, Amidine. Juvenilhormone und juvenoide synthetische Wirkstoffe, sowie Chitinsynthesehemmer wie Diarylether und Benzoylharnstoffe

Zu den Phosphor- oder Phosphorsaureestern gehören:
0-Ethyl-0-(8-chinolyl)phenyl-thiophosphat (Quintiofos),
0,0-Diethyl-0-(3-chloro-4-methyl-7-coumarinyl)-thiophosphat (Coumaphos),
0,0-Diethyl-0-phenylglyoxylonitril-oxim-thiophosphat (Phoxim),
0,0-Diethyl-0-cyanochlorbenzaldoxim-thiophosphat (Chlorphoxim),
0,0-Diethyl-0-(4-bromo-2,5-dichlorophenyl)-phosphorothionat (Bromophos-ethyl),
0,0,0',0'-Tetraethyl-S,S'-methylene-di(phosphorodithionat) (Ethion),
2,3-p-Dioxanedithiol-S,S-bis(0,0-diethylphosphorodithionat,
2-Chlor-1-(2,4-dichlorphenyl)-vinyldiethylphosphat (Chlorfenvinphos),
0, 0-Dimethyl-0-(3-methyl-4 -methylthiophenyl)-thionophosphorsäureester (Fenthion).

Zu den Carbamaten gehören:
2-Isopropoxyphenylmethylcarbamat (Propoxur),
1-Naphthyl-N-methylcarbamat (Carbaryl).

Zu den synthetischen Pyrethroiden zahlen
3-[2-(4-Chlorphenyl)-2-chlorvinyl]-2,2-dimethyl-cyclo-propancarbonsäure [(α-cyano-4-fluor-3-phenoxy)-benzyl]-ester (Flumethrin),
2,2-Dimethyl-3-(2,2-dichlorvinyl)-cyclopropancarbonsäure-α-cyano(4-fluor-3-phenoxy)-benzyl-ester (Cyfluthrin) und seine Enantiomere und Stereomere,
α-Cyano-3-phenoxybenzyl(±)-cis, trans-3-(2,2-dibromvinyl)-2,2-dimethylcyclopropancarboxylat (Deltamethrin),
2,2-Dimethyl-3-(2,2-dichlorvinyl)-cyclopropancarbonsäure-α-cyano-3-phenoxybenzylester (Cypermethrin),
3-Phenoxybenzyl(±)-cis, trans-3-(2,2-dichlorvinyl)-2,2-dimethylcyclopropancarboxylat (Permethrin),
α-(p-Cl-phenyl)-isovaleriansäure-α-cyano-3-phenoxy-benzylester (Fenvalerate),
2-Cyano-3-phenoxybenzyl-2-(2-chlor-α,α,α-trifluor-p-toluidino)-3-methylbutyrat (Fluvalinate).

Zu den Amidinen gehören:
3-Methyl-2-[2,4-dimethyl-phenylimino]-thiazolin,
2-(4-Chlor-2-methylphenylimino)-3-methylthiazolidin,
2-(4-Chlor-2-methylphenylimino)-3-(isobutyl-1-enyl)-thiazolidin
1,5-Bis-(2,4-dimethylphenyl)-3-methyl-1,3,5-triazapenta-1,4-dien (Amitraz).

Cyclische Makrolithe wie Ivermectine und Abamectine Hierzu sei beispielsweise 5-0-Dimethyl-22,23-dihydroavermectin-A₁ₐ, -22,23-dihydroavermectin B₁ₐ und 22,23-dihydroavermectin B_{b1} (vgl. beispielsweise WHO. F.A. Series 27, S. 27-73 (1991)) erwähnt. Zu den Juvenilhormonen und juvenilhormonartigen Substanzen gehören insbesondere Verbindungen der folgenden Formeln:

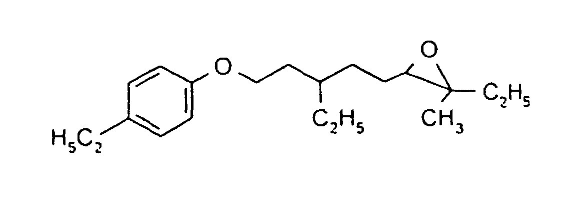

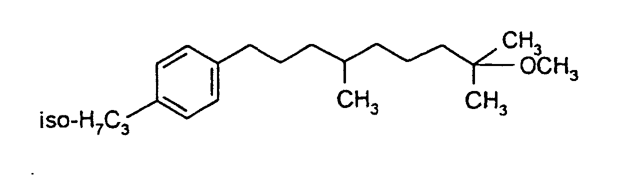

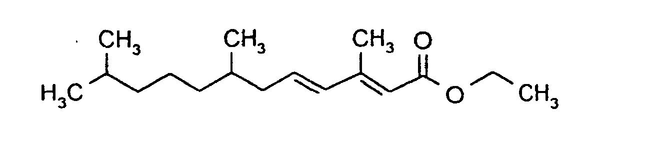

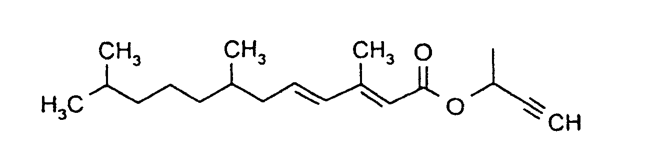

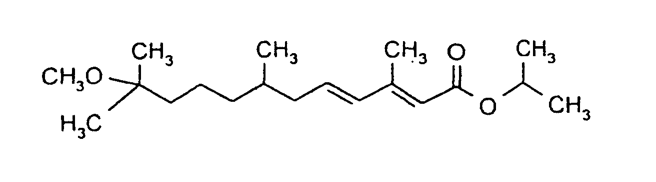

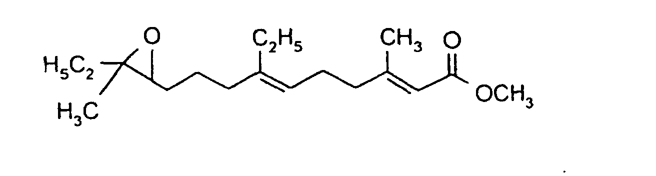

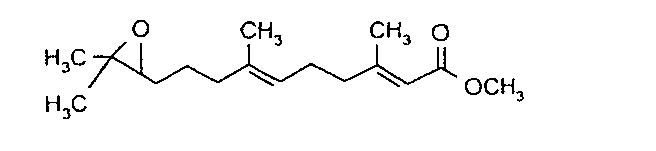

Zu den substituierten Diarylethern gehören insbesondere

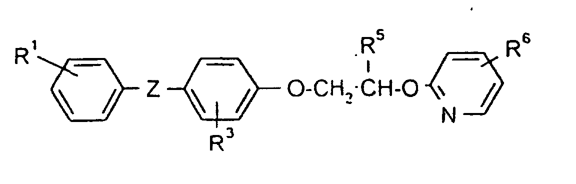

| R¹ | R³ | R⁵ | R⁶ | Z |
|---|---|---|---|---|
| H | H | CH₃ | H | O |
| H | H | CH₃ | 2-Cl | O |
| 5-F | H | CH₃ | H | O |
| H | H | CF₃ | H | O |
| H | H | C₂H₅ | H | O |
| H | H | H | H | O |
| H | H | CH₃ | H | CH₂ |
| H | H | CH₃ | H | C(CH₃)₂ |

Zu den Benzoylharnstoffen gehören Verbindungen der Formel

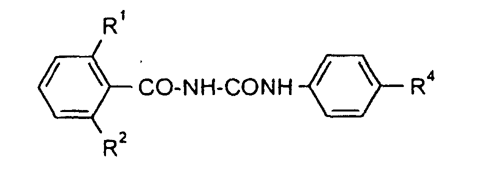

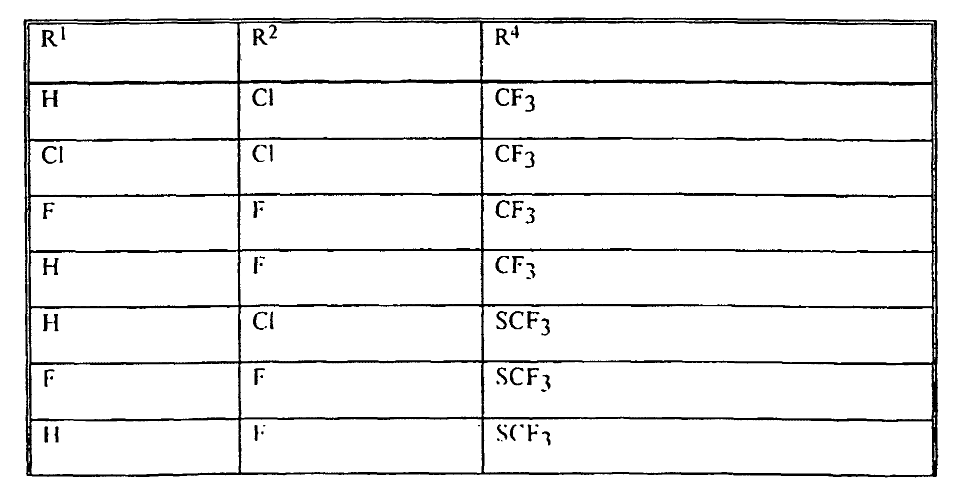

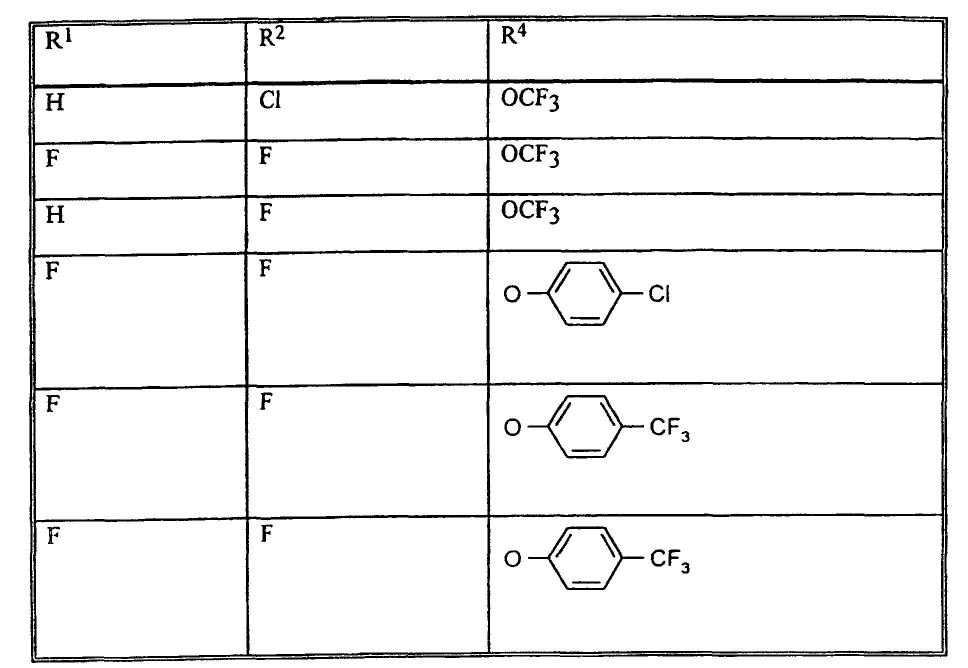

Zu den Triazinen gehören Verbindungen der Formel

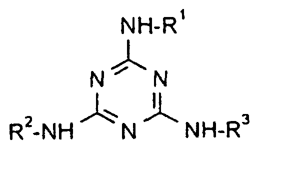

| R¹ | R² | R³ |
|---|---|---|
| Cyclopropyl | H | H |
| Cyclopropyl | H | CH₃ |
| Cyclopropyl | H | C₂H₅ |
| Cyclopropyl | H | C₃H₇-n |
| Cyclopropyl | H | C₄H₉-n |
| Cyclopropyl | H | C₅H₁₁-n |
| Cyclopropyl | H | C₆H₁₃-n |
| Cyclopropyl | H | C₇H₁₅-n |
| Cyclopropyl | H | C₈H₁₇-n |
| Cyclopropyl | H | C₁₂-H₂₅-n |
| Cyclopropyl | H | CH₂-C₄H₉-n |
| Cyclopropyl | H | CH₂CH(CH₃ )C₂H₅ |
| Cyclopropyl | H | CH₂CH=CH₂ |
| Cyclopropyl | Cl | C₂H₅ |
| Cyclopropyl | Cl | C₆H₁₃-n |
| Cyclopropyl | Cl | C₈H₁₇-n |
| Cyclopropyl | Cl | C₁₂H₂₅-n |
| Cyclopropyl | H | Cyclopropyl |
| Cyclopropyl | H | COCH₃ |
| Cyclopropyl | H | COCH₃ HCl |
| Cyclopropyl | H | COC₂H₅ HCl |
| Cyclopropyl | H | COC₂H₅ |
| Cyclopropyl | H | COC₃H₇-n |
| Cyclopropyl | H | COC₃H₇-i |
| Cyclopropyl | H | COC₄H₉-t HCl |
| Cyclopropyl | H | COC₄H₉-n |
| Cyclopropyl | H | COC₆H₁₃-n |
| Cyclopropyl | H | COC₁₁-H₂₃-n |
| Cyclopropyl | COCH₃ | COC₂H₅ |
| Cyclopropyl | COC₃H₇-n | COC₆H₁₃-n |
| Cyclopropyl | COCH3 | COC₃H₇-n |
| Cyclopropyl | COC₂H₅ | COC₃H₇-n |
| Cyclopropyl | H | COCyclopropyl |
| Cyclopropyl | COCyclopropyl | COCyclopropyl |
| Cyclopropyl | COCH₃ | COCH₃ |
| Isopropyl | H | H |
| Isopropyl | H | COCH₃ |
| Isopropyl | H | COC₃H₇-n |
| Cyclopropyl | H | CONHCH₃ |
| Cyclopropyl | H | CONHC₃H₇-i |
| Cyclopropyl | CONHCH₃ | CONHCH₃ |
| Cyclopropyl | H | SCNHCH₃ |
| Cyclopropyl | H | CONHCH₂CH=CH₂ |
| Cyclopropyl | CONHCH₂CH=CH₂ | CONHCH₂CH=CH₂ |
| Cyclopropyl | CSNHCH₃ | CSNHCH₃ |

Besonders hervorgehoben seien die weiteren Wirkstoffe mit den common names Propoxur, Cyfluthrin, Flumethrin, Pyriproxyfen, Mlethoprene, Diazinon, Amitraz, Fenthion, Levamisol und Ivermectin

In den folgenden Beispielen wird als Wirkstoff 1-[(6-Chlor-3-pyridinyl)methyl]-N-nitro-2-imidazolidinium (common name Imidacloprid) eingesetzt.

Die erfindungsgemäßen Formulierungen zeichnen sich durch ihre Stabilität bei Temperaturen im Bereich von +60°C bis -30°C aus. Aus diesem Grund sind für ihre Lagerung und für ihren Versand keine besonderen Maßnahmen erforderlich.

### Beispiel 1

| | |
|---|---|
| Imidacloprid | 10 g |
| **Wasser** | 10 g |
| Propylencarbonat | 45 g |
| Benzylalkohol | 34,8 g |
| ®Belsil DMC 6031 | 1 g |
| (Ein Polysiloxancopolymerisat der Fa. Wacker GmbH, D-81737 München) | |
| Butylhydroxytoluol | 0,2 g |

### Beispiel 2

| | |
|---|---|
| Imidacloprid | 10 g |
| Wasser | 10 g |
| n-Octylpyrrolidon-2 | 34,5 g |
| γ-Butyrolacton | 44,5 g |
| ®Belsil L 066 | 1 g |
| (Ein Polysiloxancopolymerisat der Fa. Wacker GmbH, D-81737 München) | |

### Beispiel 3

| | |
|---|---|
| Imidacloprid | 10 g |
| Wasser | 10 g |
| Ethylencarbonat | 5 g |
| Benzylalkohol | 74,8 g |
| Butylhydroxytoluol | 0,1 g |
| ®Belsil DMC 603l | 0,1 g |
| (Polysiloxancopolymerisat) | |

### Beispiel 4

| | |
|---|---|
| Imidacloprid | 10,0 g |
| Benzylalkohol | 62,4 g |
| Propylencarbonat | 17,5 g |
| Wasser | 10,0 g |
| Butylhydroxytoluol | 0,1 g |

### Beispiel 5

| | |
|---|---|
| Imidacloprid | 10,0 g |
| Benzylalkohol | 65,0 g |
| Propylencarbonat | 15,0 g |
| Isopropylmyristat | 3,8 g |
| Wasser | 6,0 g |
| Butylhydroxytoluol | 0,2 g |

### Beispiel 6

| | |
|---|---|
| Imidacloprid | 10,0 g |
| Benzylalkohol | 62,5 g |
| Propylencarbonat | 17,4 g |
| Butylhydroxytoluol | 0,1g |
| Wasser | 10,0 g |

### Beispiel 7

| | |
|---|---|
| Imidacloprid | 10,0 g |
| Benzylalkohol | 70,0 g |
| Propylencarbonat | 17,4 g |
| Wasser | 2,5 g |
| Butylhydroxytoluol | 0,1 g |

### Beispiel 8

| | |
|---|---|
| Imidacloprid | 10,0 g |
| Pyriproxyfen | 1,0 g |
| Benzylalkohol | 65,0 g |
| Wasser | 5,0 g |
| Propylencarbonat | 18,9 g |
| Butylhydroxytoluol | 0,1 g |

### Beispiel 9

| | |
|---|---|
| Imidacloprid | 10,0 g |
| Triflumuron | 2,5 g |
| Benzylalkohol | 60.0 g |
| Wasser | 7.5 g |
| Propylencarbonat | 27.5 g |

### Beispiel 10

| | |
|---|---|
| Imidacloprid | 10.0g |
| Flumetrin | 2.0 g |
| Benzylalkohol | 60.0 g |
| Propylencarbonat | 18.0 g |
| Wasser | 10.0 g |

### Beispiel 11

| | |
|---|---|
| Imidacloprid | 10.0 g |
| Benzylalkohol | 60.0 g |
| Ethylencarbonat | 10.0 g |
| Propylencarbonat | 10.0 g |
| Wasser | 9,8 g |
| Butylhydroxytoluol | 0,2 g |

### Beispiel 12

| | |
|---|---|
| Imidacloprid | 10,0 g |
| Benzylalkohol | 67,0 g |
| Propylencarbonat | 17,4 g |
| Vitamin E | 0,6 g |
| Wasser | 5,0 g |

### Anwendungsbeispiel A

4 ml der in Beispiel 1 beschriebenen Formulierung wurde einem 40 kg schweren Hund der mit Flöhen infestiert war auf den Rücken gegossen. Es wurden folgende Ergebnisse erhalten:

| Zeitraum Tag | Anzahl der Flöhe pro Hund | | % Wirkung |
|---|---|---|---|
| | unbehandelt | behandelt | |
| - 1 Infestation mit 200 Flöhen | | | |
| 0 Behandlung und Zählung | 80 | 0 | 100 |
| 5, 8 Infestation mit 200 Flöhen | | | |
| 9 Zählung | 90 | 0 | 100 |
| 15 Infestation mit 200 Flöhen | | | |
| 16 Zählung | 110 | 0 | 100 |
| 19 Infestation mit 200 Flöhen (unbehandeltes Tiere) 250 Flöhen (behandelte Tiere | | | |
| 20 Zählung | 75 | 0 | 100 |
| 26 Infestation mit 200 Flöhen | | | |
| 27 Zählung | 80 | 0 | 100 |

### Anwendungsbeispiel B

2 ml der Lösung gemäß Beispiel 4 wurden auf die Schulter eines 20 kg schweren Hundes gegeben. Das Tier wurde nach 1 und nach 6 Tagen der Behandlung mit 200 Flöhen infestiert. Jeweils am Tag 3 und am Tag 7 nach Behandlung wurden die am Hund verbliebenen Flöhe gezählt. Es konnten keine lebenden Flöhe gefunden werden. Die Wirkung war 100 %.

### Anwendungsbeispiel C

0,8 ml der Lösung gemäß Beispiel 4 wurden auf die Schulter einer - 8 kg schweren Katze gegeben. Das Tier wurde nach 3 und nach 7 Tagen der Behandlung mit 150 Flöhen infestiert. Jeweils am Tag 3 und am Tag 7 nach Behandlung wurden die am Hund verbliebenen Flöhe gezählt. Es konnten keine lebenden Flöhe gefunden werden Die Wirkung war 100 %.

### Ermittlung der Stabilität:

Zur Ermittlung der Stabilität wurden die Proben bei Temperaturen -30°C, -10°C, 0°C, +20°C, +30°C, +50°C und +60°C 4 Wochen gelagert und dann ihre Wirkstoffkonzentration mittels HPLC, Dichte, Brechungsindex, äußere Beschaffenheit und Farbe untersucht. Mit Hilfe dieser Untersuchungen konnte die Stabilität der Formulierungen belegt werden.

## Patentansprüche

1. Wasserhaltige Formulierungen zur dermalen Bekämpfung von parasitierenden Insekten an Tieren der folgenden Zusammensetzung:
a) Imidacloprid in einer Konzentration von 1 bis 20 Gew.-% bezogen auf das Gesamtgewicht der Formulierung;
b) Wasser in einer Konzentration von 2,5 bis 15 Gew.-%, bezogen auf das Gesamtgewicht der Formulierung;
c) Lösungsmittel aus der Gruppe Alkohole oder gegebenenfalls substituierten Pyrrolidone in einer Konzentration von mindestens 20 Gew.-% bezogen auf das Gesamtgewicht der Formulierung;
d) Lösungsmittel aus der Gruppe cyclischer Carbonate oder Lactone in einer Konzentration von 5 bis 50 Gew.-% bezogen auf das Gesamtgewicht der Formulierung;
e) gegebenenfalls weitere Hilfsmittel aus der Gruppe Verdickungsmittel, Spreitmittel, Farbstoffe, Antioxidantien, Treibmittel, Konservierungsstoffe, Haftmittel, Emulgatoren;
f) gegebenenfalls weiteren Wirkstoffen,
wobei die Summe von Wirkstoffen, Lösungsmitteln und Hilfsstoffen 100 Gew.-% beträgt.

2. Wasserhaltige Formulierungen gemäß Anspruch 1, in welchen abgesehen von Imidacloprid keine weiteren Wirkstoffe enthalten sind.

3. Verfahren zur Herstellung der Mittel gemäß Anspruch 1, **dadurch gekennzeichnet, dass** man den oder die Wirkstoff(e) mit Wasser und dem oder den angegebenen Lösungsmitteln zu einer homogenen Lösung vermischt und gegebenenfalls die weiteren Hilfsstoffe zusetzt.

## Claims

1. Water-containing formulations for the dermal control of parasitic insects on animals, of the following composition:
a) imidacloprid in a concentration of from 1 to 20% by weight based on the overall weight of the formulation;
b) water in a concentration of from 2.5 to 15% by weight based on the overall weight of the formulation;
c) solvents from the group alcohols or optionally substituted pyrrolidones in a concentration of at least 20 % by weight based on the overall weight of the formulation;
d) solvents from the group of the cyclic carbonates or lactones in a concentration of from 5 to 50% by weight based on the overall weight of the formulation;
e) if desired, further auxiliaries from the group thickeners, spreading agents, colorants, antioxidants, propellants, preservatives, adhesives, emulsifiers;
f) if desired, further active compounds, the sum of active compounds, solvents and auxiliaries being 100% by weight.

2. Water-containing formulations according to Claim 1, which, apart from imidacloprid, do not contain any further active compounds.

3. Process for preparing the compositions according to Claim 1, **characterized in that** the active compound(s) is/are mixed with water and the stated solvent(s) to give a homogeneous solution and, if appropriate, the other auxiliaries are added.

## Revendications

1. Formulations aqueuses pour la lutte dermique contre des insectes parasites sur des animaux, de la composition suivante :
a) l'imidaclopride en une concentration allant de 1 à 20% en poids, sur base du poids total de la formulation ;
b) de l'eau en une concentration allant de 2,5 à 15% en poids, sur base du poids total de la formulation ;
c) un solvant du groupe des alcools ou de la pyrrolidone le cas échéant substituée en une concentration d'au moins 20% en poids, sur base du poids total de la formulation ;
d) un solvant du groupe des carbonates cycliques ou lactones en une concentration allant de 5 à 50% en poids, sur base du poids total de la formulation ;
e) le cas échéant, d'autres auxiliaires du groupe des agents épaississants, des agents d'étalement, des colorants, des antioxydants, dès propulseurs, des conservateurs, des agents d'adhérence, des émulsionnants ;
f) le cas échéant, d'autres agents actifs,
où la somme des agents actifs, solvants et auxiliaires s'élève à 100%.

2. Formulations aqueuses selon la revendication 1, dans lesquelles à l'exclusion de l'imidaclopride, il n'y a aucun autre agent actif.

3. Procédé de préparation de l'agent selon la revendication 1, **caractérisé en ce que** l'on mélange le ou les agents actifs avec l'eau et le ou les solvants indiqués en une solution homogène et on y ajoute, le cas échéant, les autres auxiliaires.
